# EUROPEAN PATENT APPLICATION

(11) **EP 1 757 969 A1**
(43) Date of publication of application: **28.02.2007**
(21) Application number: 06016708.7
(22) Date of filing: 10.08.2006
(51) Int. Cl.: G02B 21/00

(54) **Microscope moving unit and microscope apparatus**

(30) Priority: 24.08.2005 JP 2005242360; 24.08.2005 JP 2005242361
(71) Applicant: Olympus Corporation, Tokyo 151-0072 (JP)
(72) Inventor: Honda, Susumu, Hachioji-shi Tokyo 151-0072 (JP); Kawano, Yoshihiro, Hachioji-shi Tokyo 192-0916 (JP)
(74) Representative: von Hellfeld, Axel

(57) **Abstract**

A microscope moving unit (2) is provided, the microscope moving unit being capable of preventing an increase in size and a decrease in operation speed of a microscope apparatus (1) by reducing the size of the movable parts, improving the operability of the objective lens (7) by providing a large space around the objective lens, and allowing a specimen (A) to be observed at various positions without affecting the specimen. The microscope moving unit (2) includes an arm (17) that is rotatable around an optical axis of a laser beam (L) guided from a laser light source (3) and that supports a microscope main body (4) on the side closer to a tip of the arm, the microscope main body having an objective lens (7), and a rotatable deflecting member (22), which is fixed to the arm, configured to deflect the laser beam and emit the deflected laser beam towards the microscope main body at the side closer to the tip of the arm.

## Description

### BACKGROUND OF THE INVENTION

### 1. FIELD OF THE INVENTION

The present invention relates to microscope moving units and microscope apparatuses.

This application is based on Japanese Patent Application Nos. 2005-242360 and 2005-242361, the content of which is incorporated herein by reference.

### 2. DESCRIPTION OF RELATED ART

Microscope apparatuses of the related art are used to observe a specimen by moving an objective lens close to the specimen, which is placed on a stage.

The stage of such a known microscope apparatus is movable horizontally in two directions (X and Y directions). To observe different areas on the specimen, the stage is operated to move the specimen in a direction that brings to be observed to a position where it intersects with the optical axis of the objective lens.

However, if the specimen placed on the stage is, for example, a living cell floating in a culture medium, the specimen will be accelerated by the movement of the stage. Therefore, the specimen might move in the culture medium, or the specimen may be deformed. If the specimen is a small laboratory animal having a relatively large size, the specimen might be shifted by a lateral force applied thereto.

To overcome these problems, a microscope apparatus having a fixed stage and a movable microscope main body, such as the microscope apparatus described in Japanese Unexamined Patent Application Publication No. 2000-88751, has been proposed.

Since the stage of such a microscope apparatus is fixed, different areas of a specimen can be observed without affecting the specimen placed on the stage.

However, since the microscope main body of the microscope apparatus according to Japanese Unexamined Patent Application Publication No. 2000-88751 is fixed to a slider that is movable along guiding rails disposed below the stage, the movable portion of the microscopic apparatus is large. Therefore, to achieve satisfactory rigidity, a strong frame is provided, causing the overall size of the microscope apparatus increase. Such a large microscope apparatus may cause problems such as an increase in the space required for installation of the apparatus and a reduction in operating speed. To observe a relatively large specimen, a large space has to be provided around the objective lens to allow the objective lens to move freely. Moreover, if the specimen is relatively large, the specimen may have to be displaced and/or rotated on the stage to be observed from different directions.

### BRIEF SUMMARY OF THE INVENTION

The present invention has been conceived in light of the problems described above. Accordingly, with a microscope moving unit and a microscope apparatus according to embodiments of the present invention, it is possible to prevent an increase in size and a decrease in operation speed of the apparatus by reducing the size of the movable parts, to improve the operability of the objective lens by providing a large space around the objective lens, and to enable observation of a specimen from various different angles.

To achieve the above-described object, the present invention provides the following solutions.

The present invention provides a microscope moving unit including an arm that is rotatable around an optical axis of a laser beam guided from a laser light source and that supports a microscope main body at the side closer to a tip of the arm, the microscope main body having an objective lens, and a rotatable deflecting member, which is fixed to the arm, configured to deflect the laser beam and emit the deflected laser beam towards the microscope main body at the side closer to the tip of the arm.

According to the present invention, the laser beam emitted from the laser light source is transmitted through the microscope moving unit and is incident on the microscope main body. The laser beam incident on the microscope main body is transmitted through the objective lens and is incident on a specimen. Light, such as fluorescence, generated at the specimen is focused by the objective lens and is detected. In this case, to change the observation angle of the specimen, the arm is turned. The laser beam guided from the laser light source is emitted along the rotational axis of the arm. Since a rotatable deflecting member is fixed to the arm, the laser beam emitted along the rotational axis of the arm is deflected at the rotatable deflecting member and is incident on the microscope main body disposed on the side closer to a tip of the arm.

At this time, since the arm is fixed to the rotatable deflecting member, when the arm is operated, the rotatable deflecting member is turned together with the microscope main body. Therefore, regardless of the rotation angle of the arm, the incident laser beam is deflected at the rotatable deflecting member and is always oriented towards the microscope main body.

In other words, according to the present invention, the angle of the microscope main body can be changed without moving the specimen so as to observe the specimen from different angles. As a result, specimens, such as living cells and small laboratory animals, can be observed from different angles while keeping them static, without applying a lateral force.

In such a case, the laser beam does not have to be transmitted through an optical element, such as an optical fiber, since the laser beam is always guided to the microscope main body by the rotatable deflecting member. Therefore, for example, even when an ultrashort pulsed laser light source is used as the laser light source, group velocity dispersion of the laser beam and an increase in pulse width are prevented until the laser beam reaches the specimen. As a result, a large group velocity dispersion compensator is not required, and a multiphoton excitation effect is efficiently generated to obtain a clear multiphoton fluorescence image.

According to the present invention, the microscope moving unit may include a moving member, which is disposed on the side closer to the tip of the arm, configured to move the microscope main body with respect to a specimen in a direction parallel to the optical axis of the laser beam guided from the laser light source and in a direction orthogonal to the optical axis of the objective lens. A deflecting member configured to deflect the laser beam and emit the deflected laser beam towards the microscope main body may be fixed to the moving member.

In this way, to change the incident position of the laser beam on the specimen, the moving member can be moved to move the microscope main body parallel to the optical axis of the laser beam and orthogonal to the optical axis of the objective lens. At this time, by moving the moving member, the deflecting member is moved together with the microscope main body along the optical axis of the laser beam because the deflecting member is fixed to the moving member. Therefore, regardless of the position of the moving member, the laser beam is deflected at the deflecting member and is always oriented toward the microscope main body.

According to the present invention, a different area of the specimen can be observed by moving the microscope main body while maintaining the angle of the microscope main body, without moving the specimen.

According to the present invention, it is preferable that the microscope moving unit include a first moving member, which is disposed on the side closer to the tip of the arm, movable in a first direction orthogonal to the optical axis of the objective lens; a second moving member, which is disposed on the first moving member and has the microscope main body fixed thereto, movable in a second direction orthogonal to the optical axis of the objective lens and orthogonal to the first direction; a first deflecting member, which is fixed to the first moving member, configured to deflect a laser beam emitted along the first direction and emit the deflected laser beam in the second direction; and a second deflecting member, which is fixed to the second moving member, configured to deflect the laser beam emitted in the second direction and emit the deflected laser beam towards the microscope main body.

In this way, when the first moving member is moved in the first direction, the second moving member mounted on the first moving member and the microscope main body attached to the second moving member are moved in the first direction orthogonal to the optical axis of the objective lens. Since the laser beam is emitted in the first direction and the first deflecting member is fixed to the first moving member, the emitted laser beam is deflected in the second direction at the first deflecting member, regardless of the position of the first moving member.

By moving the second moving member in the second direction with respect to the first moving member, the microscope main body attached to the second moving member is moved in the second direction orthogonal to the optical axis of the objective lens. Since a laser beam is emitted in the second direction from the first deflecting member, the second deflecting member deflects the laser beam toward the objective lens, regardless of the position of the second moving member.

Therefore, according to the present invention, the objective lens can be positioned at a prescribed position in the plane orthogonal to the optical axis of the objective lens by adjusting the positions of the first and second moving members. Accordingly, the specimen can be observed two-dimensionally while the angle of the microscope main body is fixed and without applying lateral acceleration.

According to the present invention, the microscope may include a third moving member configured to movably hold the first moving member and move the first moving member in the direction of the optical axis of the objective lens and a third deflecting member, which is fixed to the third moving member, configured to deflect a laser beam and emit the deflected laser beam in the first direction.

In this way, the objective lens can be moved parallel to the optical axis thereof without being required to transmit the laser beam through an optical element, such as an optical fiber.

The present invention provides a laser microscope apparatus including a laser light source configured to emit a laser beam, a microscope main body configured to emit the laser beam generated at the laser light source onto a specimen and detect light emitted from the specimen, and one of the microscope moving units described above.

According to the present invention, by moving the microscope moving unit, the angle of the microscope main body can be changed with respect to a specimen. In the microscope moving unit, the laser beam from the laser light source is deflected towards the microscope main body at the rotatable deflecting member that moves together with the microscope main body. Thus, a laser beam can be emitted toward a specimen for microscopy, regardless of the angle of the microscope main body. Therefore, the observation direction can be changed without moving and/or applying acceleration to the specimen, and the specimen can be observed from various different angles.

The present invention provides a laser microscope apparatus including a laser light source configured to emit a laser beam; a microscope main body, which is disposed with an objective lens thereof facing downwards, configured to emit the laser beam generated at the laser light source onto a specimen and detect light emitted from the specimen; and a microscope moving unit configured to support the microscope main body in a suspended position and to move the microscope main body with respect to the specimen. The microscope moving unit includes a moving member configured to move the microscope main body with respect to the specimen in a direction parallel to the optical axis of the laser beam guided from the laser light source and in a direction orthogonal to the optical axis of the objective lens, and a deflecting member, which is fixed to the moving member, configured to deflect the laser beam and emit the deflected laser beam towards the microscope main body.

According to the present invention, the laser beam emitted from the laser light source is transmitted through the microscope moving unit and is incident on the microscope main body. The laser beam incident on the microscope main body is transmitted through the objective lens and is incident on the specimen. Light, such as fluorescence, generated at the specimen is focused by the objective lens and is detected. In this case, to change the incident position of the laser beam on the specimen, the moving member is moved to move the microscope main body parallel to the optical axis of the laser beam and orthogonal to the optical axis of the objective lens.

At this time, the deflecting member is moved together with the microscope main body when the moving member is moved since the deflecting member is fixed to the moving member. Since both the deflecting member and the moving member are moved parallel to the optical axis of the laser beam, the laser beam is deflected at the deflecting member, regardless of the position of the deflecting member, and is always oriented towards the microscope main body.

Therefore, according to the present invention, different positions of the specimen can be observed by moving the microscope main body, without moving the specimen. As a result, specimens, such as living cells and small laboratory animals, can be observed from different angles in a static state without applying a lateral force.

In such a case, the laser beam does not have to be transmitted through an optical element, such as an optical fiber, since the laser beam is always guided to the microscope main body by the rotatable deflecting member. Therefore, for example, even when an ultrashort pulsed laser light source is used as the laser light source, group velocity dispersion of the laser beam and an increase in pulse width are prevented until the laser beam reaches the specimen. As a result, a large-scale group velocity dispersion compensator is not required, and a multiphoton excitation effect is efficiently generated to obtain a clear multiphoton fluorescence image.

According to the present invention, the moving part including the microscope main body can be minimized because the moving unit supports the microscope main body in a suspended state. As a result, an increase in the size of the microscope apparatus can be prevented and the moving part can be moved quickly and accurately. By suspending the microscope main body, sufficient space can be provided around the objective lens disposed at the tip of the microscope main body, and manipulation of relatively large specimens becomes easy.

According to the present invention, a laser microscope apparatus according includes a first moving member movable in a first direction orthogonal to the optical axis of the objective lens and a second moving member, which is disposed on the first moving member and has the microscope main body fixed thereto, movable in a second direction orthogonal to the optical axis of the objective lens and orthogonal to the first direction. The deflecting member includes a first deflecting member, which is fixed to the first moving member, configured to deflect the laser beam and emit the deflected laser beam in the second direction and a second deflecting member, which is fixed to the second moving member, configured to deflect the laser emitted in the second direction. It is preferable that the laser beam from the laser light source be emitted in the first direction and be incident on the first deflecting member.

In this way, when the first moving member is moved in the first direction, the second moving member mounted on the first moving member and the microscope main body attached to the second moving member are moved in the first direction orthogonal to the optical axis of the objective lens. Since the laser beam is emitted in the first direction and the first deflecting member is fixed to the first moving member, the emitted laser beam is deflected in the second direction at the first deflecting member, regardless of the position of the first moving member.

By moving the second moving member in the second direction with respect to the first moving member, the microscope main body attached to the second moving member is moved in the second direction orthogonal to the optical axis of the objective lens. Since a laser beam is emitted in the second direction from the first deflecting member, the second deflecting member deflects the laser beam toward the objective lens, regardless of the position of the second moving member.

Therefore, according to the present invention, the objective lens can be positioned at a prescribed position in the plane orthogonal to the optical axis of the objective lens by adjusting the positions of the first and second moving members. Accordingly, the specimen can be observed two-dimensionally while the angle of the microscope main body is fixed and without applying lateral acceleration.

According to the present invention, the microscope may include a third moving member configured to movably hold the first moving member and move the first moving member in the direction of the optical axis of the objective lens and a third deflecting member, which is fixed to the third moving member, configured to deflect a laser beam and emit the deflected laser beam in the first direction.

In this way, the objective lens can be moved parallel to the optical axis thereof without being required to transmit the laser beam through an optical element, such as an optical fiber.

According to the present invention, by minimizing the side of the moving part, an increase in the size of the microscope apparatus can be prevented and the moving part can be moved quickly and accurately. By providing a large space around the objective lens, relatively large specimens can be manipulated easily and the ease of moving the objective lens with respect to the specimen can be increased. The specimen can be observed, without being affected, from various different angles and at various different positions. Accordingly, fragile specimens, such as living cells, and relative large specimens, such as small laboratory animals, can be observed from various directions without being affected.

### BRIEF DESCRIPTION OF THE SEVERAL VIEWS OF THE DRAWINGS

Fig. 1 is a schematic view illustrating the overall structure of a microscope apparatus according to an embodiment of the present invention.
Fig. 2 is a perspective view illustrating the operation of a moving unit of the microscope apparatus illustrated in Fig. 1.
Fig. 3 is a schematic view illustrating the overall structure of a modification of the microscope apparatus illustrated in Fig. 1.
Fig. 4 is a perspective view illustrating the operation of a moving unit of the microscope apparatus illustrated in Fig. 3.

### DETAILED DESCRIPTION OF THE INVENTION

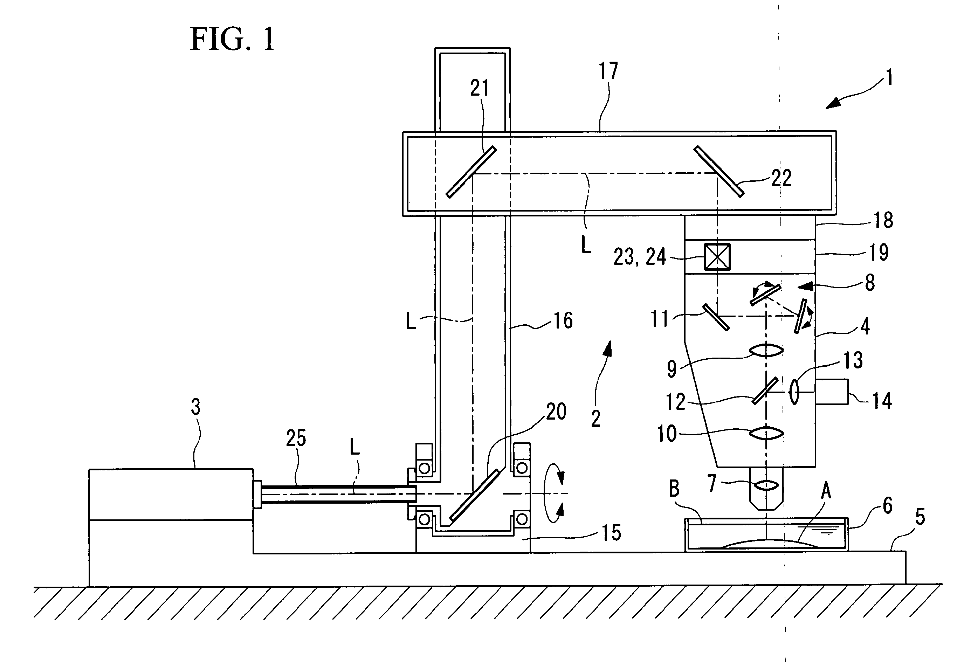

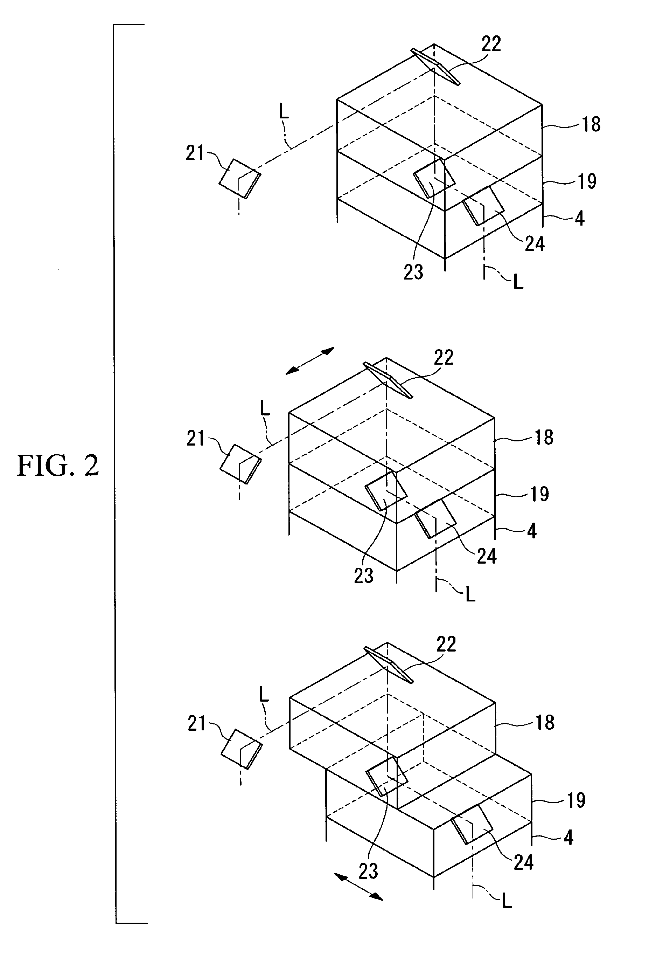

A microscope apparatus 1 and a moving unit (microscope moving unit) 2 according to a first embodiment will be described below with reference to Figs. 1 and 2.

As shown in Fig. 1, the microscope apparatus 1 according to this embodiment includes a laser light source 3, a microscope main body 4 for observing light emitted from a specimen A when a laser beam L is incident on the specimen A, and the moving unit 2 for making the laser beam L from the laser light source 3 enter the microscope main body 4.

The laser light source 3 is an ultrashort pulse laser light source, e.g., a titanium sapphire laser, capable of generating a near-infrared pulsed laser beam having a pulse width of about 100 femtoseconds (fs) and a wavelength of about 800 nm. The laser light source 3 is fixed to a substantially horizontal base plate 5 and emits a laser beam L in a horizontal direction. A cylindrical cover 25 capable of transmitting the laser beam L is provided.

The specimen A, for example, is a living cell disposed in a culture medium B in a petri dish 6.

An objective lens 7 that opposes the specimen A is provided at the tip of the microscope main body 4. The microscope main body 4 scans the laser beam L transmitted from the moving unit 2 in a two-dimensional manner. The microscope main body 4 includes, for example, a scanning unit 8, such as proximate galvanometer mirrors, a pupil projection lens 9 configured to form an intermediate image by focusing the laser beam L two-dimensionally scanned by the scanning unit 8, and an imaging lens 10 configured to converge the laser beam L that is focused to form the intermediate image at the objective lens 7. In addition, as shown in the drawing, a mirror 11 is provided.

A dichroic mirror 12 configured to split off multiphoton fluorescence, a condenser lens 13 configured to focus the split-off multiphoton fluorescence, and a light detecting unit 14 configured to detect the focused multiphoton fluorescence are provided between the imaging lens 10 and the pupil projection lens 9. Information on the multiphoton fluorescence detected by the light detecting unit 14 is sent to an image processing apparatus (not shown) and is displayed on a monitor, for example. The light detecting unit 14 is, for example, a photomultiplier tube.

The moving unit 2 is supported by a bracket 15 fixed to the base plate 5 holding the laser light source 3 and is rotatable around the optical axis of the laser beam L from the laser light source 3. The moving unit 2 includes a first arm 16, a second arm 17, a first slider (first moving member) 18, and a second slider (second moving member) 19. The first arm 16 extends along a vertical plane; the second arm 17 extends in the horizontal direction, which is the direction orthogonal to the longitudinal direction of the first arm 16, and is supported in such a manner that it is movable along the longitudinal direction of the first arm 16; the first slider 18 is attached to the second arm 17 and is supported in such a manner that it is movable in the longitudinal direction of the second arm 17; and the second slider 19 is attached to the first slider 18, is supported in such a manner that it is movable in the direction orthogonal to the direction in which the first slider 18 moves, and holds the microscope main body 4 in a suspended position.

The first arm 16 is rotated around a shaft of a motor, not shown in the drawings. The second arm 17, the first slider 18, and the second slider 19 are movably supported by, for example, a known linear guide (not shown in the drawings) are linearly driven by, for example, a known ball screw (not shown in the drawings) and are capable of being held at predetermined positions.

A first mirror (deflecting member) 20 is disposed on the rotational axis of the first arm 16. The first mirror 20 reflects the laser beam L emitted from the laser light source 3 along the axis and directs the laser beam L along the longitudinal direction of the first arm 16. The first mirror 20 is fixed and rotated together with the first arm 16 to constantly reflect the laser beam L from the laser light source 3 along the longitudinal direction of the first arm 16.

A second mirror (deflecting member) 21 is disposed on the second arm 17, on the optical axis of the laser beam L reflected by the first mirror 20. The second mirror 21 is fixed to the second arm 17, which is linearly movable in the longitudinal direction of the first arm 16. Together with the second arm 17, the second mirror 21 is linearly movable along the optical axis of the laser beam L from the first mirror 20. The second mirror 21 reflects the laser beam L along the longitudinal direction of the second arm 17. In this way, regardless of the position of the second arm 17 with respect to the first arm 16, the second mirror 21 is capable of constantly directing the laser beam L from the first mirror 20 in the longitudinal direction of the second arm 17.

As shown in Fig. 2, a third mirror (deflecting member) 22 and a fourth mirror (deflecting member) 23 are fixed to the first slider 18. The third mirror 22 is disposed at a position opposite to the second mirror 21 fixed to the second arm 17 and receives the laser beam L reflected at the second mirror 21. The third mirror 22 reflects the laser beam L in a direction orthogonal to the direction in which the laser beam L is incident on the third mirror 22 in order to transmit the laser beam L to the second slider 19. The fourth mirror 23 reflects the laser beam L reflected at the third mirror 22 in the direction in which the second slider 19 moves.

Since the third mirror 22 and the fourth mirror 23 are fixed to the first slider 18, the third mirror 22 and the fourth mirror 23 move together with the first slider 18 when the first slider 18 is moved in the longitudinal direction of the second arm 17. Therefore, regardless of the position of the first slider 18, the third mirror 22 and the fourth mirror 23 are capable of constantly directing the laser beam L reflected at the second mirror 21 in the moving direction of the second slider 19.

As shown in Fig. 2, a fifth mirror (deflecting member) 24 is fixed to the second slider 19. The fifth mirror 24 is disposed on the optical axis of the laser beam L reflected at the fourth mirror 23 and is capable of reflecting the laser beam L in order to direct the laser beam L into the microscope main body 4. Since the fifth mirror 24 is fixed to the second slider 19, the fifth mirror 24 moves together with the second slider 19. Therefore, the fifth mirror 24 is capable of constantly directing the laser beam L into the microscope main body 4.

The operation of the microscope apparatus 1 according to this embodiment, having the above-described structure, will be described below.

To carry out multiphoton fluoroscopy of the specimen A from directly above using the microscope apparatus 1 according to this embodiment, the first arm 16 is disposed along the vertical direction. In this way, the second arm 17 is extended horizontally above the specimen A; the first slider 18, the second slider 19, and the microscope main body 4 are disposed directly below the second arm 17; and the objective lens 7 is disposed on the lower area of the microscope main body 4 facing directly below. According to this configuration, the first slider 18 and the second slider 19 are moved to adjust the horizontal position of the objective lens 7 with respect to the specimen A, whereas the second arm 17 is moved with respect to the first arm 16 to adjust the vertical position of the objective lens 7 with respect to the specimen A. By operating the laser light source 3, the laser beam L is horizontally emitted into the first arm 16 along the rotational axis of the first arm 16.

The first mirror 20 disposed on the rotational axis of the first arm 16 reflects the laser beam L in the longitudinal direction of the first arm 16. Since the second arm 17 is disposed at the end of the first arm 16 and the second mirror 21 is disposed on the optical axis of the laser beam L, the laser beam L is reflected at the second mirror 21 and is directed in the longitudinal direction of the second arm 17.

Since the third mirror 22 fixed to the first slider 18 is disposed at the end of the second arm 17, the laser beam L, after being reflected at the third mirror 22, is reflected at the fourth mirror 23 fixed to the first slider 18 and is directed in the moving direction of the second slider 19. Since the fifth mirror 24 fixed to the second slider 19 is disposed on the optical axis of the laser beam L, the laser beam L is reflected at the fifth mirror 24 and enters the microscope main body 4.

The laser beam L entering the microscope main body 4 is scanned two-dimensionally by operating the scanning unit 8 and is incident on the specimen A through the pupil projection lens 9, the imaging lens 10, and the objective lens 7. The specimen A emits fluorescence F from a point where the laser beam L is focused at a predetermined depth due to a multiphoton excitation effect. The emitted fluorescence F returns along the same light path through the objective lens 7 and the imaging lens 10 and is split off from the light path at the dichroic mirror 12. The split off fluorescence F is focused by the condenser lens 13 and is detected at the light detecting unit 14. A two-dimensional image can be formed on the basis of angular information of the galvanometer mirrors constituting the scanning unit 8 and information about the amount of multiphoton fluorescence detected at the light detecting unit 14.

At this time, the examination site of the specimen A can be changed by moving the first slider 18 with respect to the second arm 17 to horizontally move the microscope main body 4 in the longitudinal direction of the second arm 17. Furthermore, by moving the second slider 19 with respect to the first slider 18, the microscope main body 4 can be moved in the direction orthogonal to the longitudinal direction of the second arm 17. In this way, the examination site can be moved two-dimensionally in the horizontal direction to another predetermined position while continuously observing the specimen A from directly above.

The observation height of the specimen A can be changed by moving the second arm 17 with respect to the first arm 16 to vertically move the microscope main body 4 in order to vertically move the focal point of the laser beam L focused by the objective lens 7.

To change the observation angle of the specimen A, the first arm 16 is rotated with respect to the bracket 15 fixed to the base plate 5. In this way, the inclination angle of the microscope main body 4 is changed to alter the observation angle of the specimen A.

In the microscope apparatus 1 according to the first embodiment, the moving unit 2 supports the microscope main body 4 in a suspended position with the objective lens 7 facing downwards. Therefore, by moving only the microscope main body 4, which is interposed between the moving unit 2 and the specimen A, the examination site of the specimen A can be changed. Accordingly, the size of the movable part can be reduced by providing a small-sized microscope main body 4. As a result, the size of the entire apparatus can be reduced. Moreover, a large space can be provided around the objective lens 7. In this way, even when the specimen A is relatively large, sufficient space for manipulating the specimen A is provided, thus improving the ease of use of the apparatus.

The examination site and the observation angle of the specimen A can be changed by moving the microscope main body 4 without moving the specimen A nor applying horizontal acceleration to the specimen A. Accordingly, when observing a specimen that should be handled with care, such as a living cell, or a specimen that has a great mass and might be substantially displaced when horizontal acceleration is applied, different areas of the specimen can be freely observed from various observation angles.

The microscope apparatus 1 according to this embodiment relays the laser beam L using the mirrors 20 to 24, without using optical fiber, and is capable of freely changing the position of the microscope main body 4 in accordance with the examination site and the observation angle. Therefore, if an ultrashort pulsed laser beam is used as the laser beam L, according to this embodiment, the pulse width can be prevented from increasing due to group velocity dispersion before the laser beam L is incident on the specimen A. Accordingly, large-scale components, such as a group velocity dispersion compensator, are not required, and the overall size of the apparatus, including the laser light source 3, can be reduced.

Since it is possible to prevent the pulse width of the ultrashort pulsed laser beam L increasing, the multiphoton excitation effect efficiently occurs at the focal point in the specimen A, and a high-resolution multiphoton fluorescence image can be obtained.

When the laser beam L is transmitted through an optical fiber, the intensity of the laser beam L incident on the specimen A changes due to movement of the optical fiber. Therefore, the transmittance of the laser beam L is unstable. However, according to this embodiment, the laser beam L is incident on the specimen A with a uniform intensity. Thus, unstable transmittance is prevented.

In the microscope apparatus 1 according to this embodiment, the laser beam L from the laser light source 3 is guided to the moving unit 2 through the cover 25, the first arm 16, and the second arm 17. Therefore, a special member for blocking the laser beam L to prevent it from escaping outside is not required. Thus, the structure of the apparatus can be simplified.

The microscope apparatus 1 according to the embodiment of the present invention is most suitable for multiphoton excitation observation. However, the use of the microscope apparatus 1 is not limited, and the microscope apparatus 1 may be included in a single-photon-excitation fluoroscopy apparatus. In such a case, instead of the light detecting unit 14 provided on the microscope main body 4, a dichroic mirror may be disposed at a fixed optical path between the laser light source 3 and the first arm 16 so as to split off the fluorescence F. In this way, the size and the weight of the microscope main body 4 can be reduced even more, and the ease of use of the apparatus can be improved.

According to this embodiment, mirrors for reflecting the laser beam L are provided as deflecting members. Instead of this, however, any deflecting member, such as prisms, may be provided.

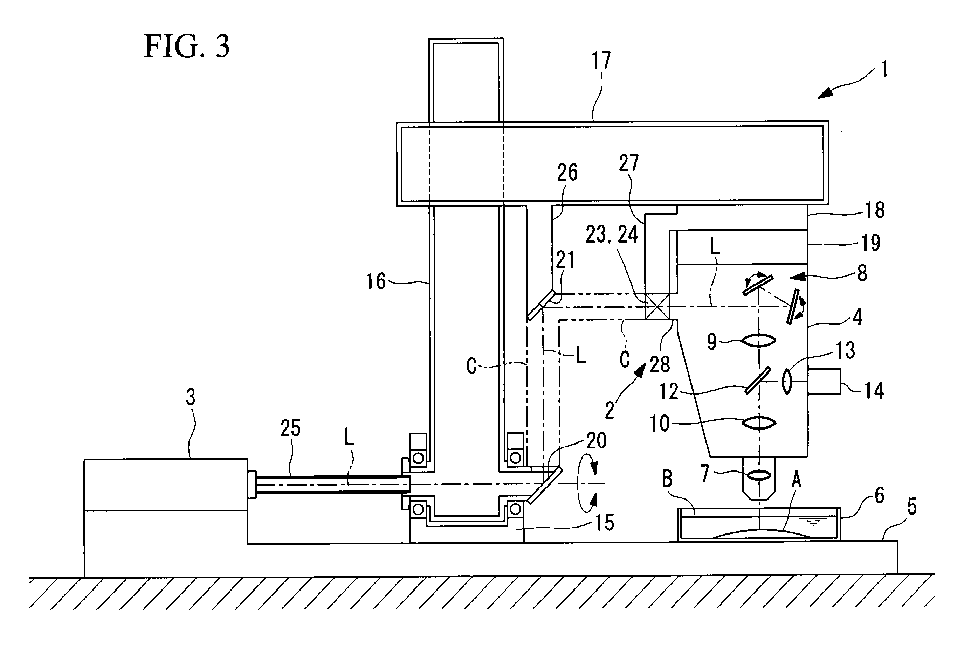

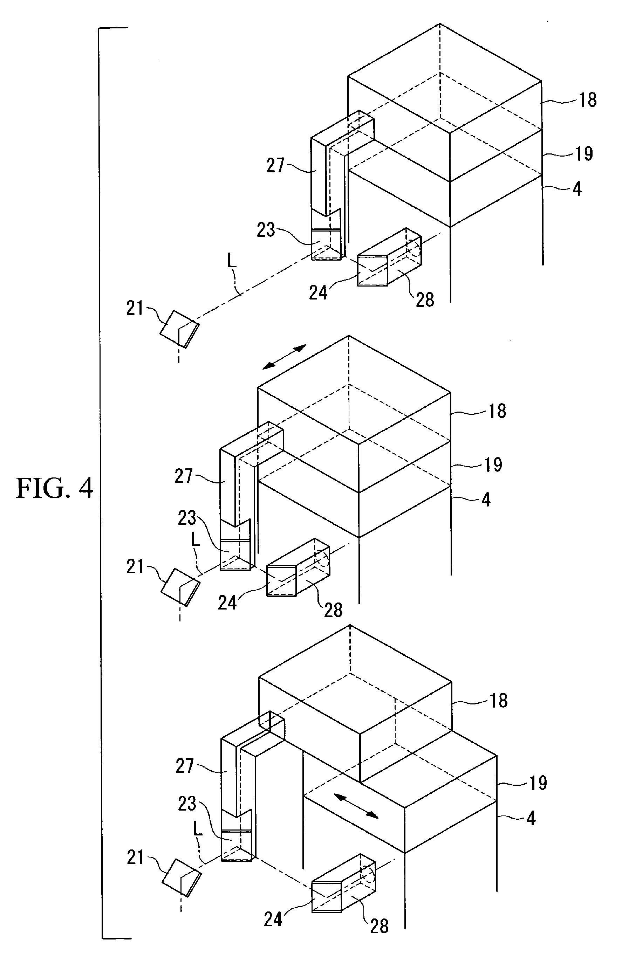

As described above, in the microscope apparatus 1 according to this embodiment, the laser beam L is guided from the laser light source 3 to the moving unit 2 through the cover 25, the first arm 16, and the second arm 17. However, as shown in Figs. 3 and 4, the laser beam L may be guided along the exterior of the arms. These drawings also illustrate a support 26 protruding outwards from the second arm 17 and configured to support the second mirror 21, a support 27 protruding outwards from the first slider 18 and configured to support the fourth mirror 23, and a support 28 protruding outwards from the microscope main body 4 fixed to the second slider 19 and configured to support the fifth mirror 24.

In this case, since the laser beam emitted from the laser light source 3 is transmitted along an external light path, the entire microscope apparatus 1 should be light shielded or a retractable bellows-like light-blocking member should be provided at the position indicated by the dashed-dotted line C in Fig. 3.

Thus, compared with the microscope apparatus 1 illustrated in Fig. 1, the third mirror 22 and the display element 11 may be omitted, allowing the structure to be simplified even more.

## Claims

1. A microscope moving unit comprising:
an arm rotatable around an optical axis of a laser beam guided from a laser light source, the arm supporting a microscope main body on the side closer to a tip of the arm, the microscope main body having an objective lens; and
a rotatable deflecting member configured to deflect the laser beam and emit the deflected laser beam towards the microscope main body at the side closer to the tip of the arm, the rotatable deflecting member being fixed to the arm.

2. The microscope moving unit according to Claim 1 further comprising:
a moving member configured to move the microscope main body with respect to a specimen in a direction parallel to the optical axis of the laser beam guided from the laser light source and in a direction orthogonal to the optical axis of the objective lens, the moving member being disposed on the side closer to the tip of the arm,
wherein a deflecting member configured to deflect the laser beam and emit the deflected laser beam towards the microscope main body is fixed to the moving member.

3. The microscope moving unit according to Claim 1 further comprising:
a first moving member movable in a first direction orthogonal to the optical axis of the objective lens, the first moving member being disposed on the side closer to the tip of the arm;
a second moving member movable in a second direction orthogonal to the optical axis of the objective lens and orthogonal to the first direction, the second moving member being disposed on the first moving member and having the microscope main body fixed thereto;
a first deflecting member configured to deflect a laser beam emitted along the first direction and emit the deflected laser beam in the second direction, the first deflecting member being fixed to the first moving member; and
a second deflecting member configured to deflect the laser beam emitted in the second direction and emit the deflected laser beam towards the microscope main body, the second deflecting member being fixed to the second moving member.

4. The microscope moving unit according to Claim 3 further comprising:
a third moving member configured to movably hold the first moving member and move the first moving member in the direction of the optical axis of the objective lens; and
a third deflecting member configured to deflect a laser beam and emit the deflected laser beam in the first direction, the third deflecting member being fixed to the third moving member.

5. A laser microscope apparatus comprising:
a laser light source configured to emit a laser beam;
a microscope main body configured to emit the laser beam generated at the laser light source onto a specimen and detect light emitted from the specimen; and
a microscope moving unit according to one of Claims 1 to 4.

6. A laser microscope apparatus comprising:
a laser light source configured to emit a laser beam;
a microscope main body configured to emit the laser beam generated at the laser light source onto a specimen and detect light emitted from the specimen, the microscope main body being disposed with an objective lens thereof facing downwards; and
a microscope moving unit configured to support the microscope main body in a suspended position and to move the microscope main body with respect to the specimen,
wherein the microscope moving unit includes,
a moving member configured to move the microscope main body with respect to the specimen in a direction parallel to the optical axis of the laser beam guided from the laser light source and in a direction orthogonal to the optical axis of the objective lens, and
a deflecting member configured to deflect the laser beam and emit the deflected laser beam towards the microscope main body, the deflecting member being fixed to the moving member.

7. The laser microscope apparatus according to Claim 6 further comprising:
a first moving member movable in a first direction orthogonal to the optical axis of the objective lens; and
a second moving member movable in a second direction orthogonal to the optical axis of the objective lens and orthogonal to the first direction, the second moving member being disposed on the first moving member and having the microscope main body fixed thereto,
wherein the deflecting member includes,
a first deflecting member configured to deflect the laser beam and emit the deflected laser beam in the second direction, the first deflecting member being fixed to the first moving member, and
a second deflecting member configured to deflect the laser emitted in the second direction, the second deflecting member being fixed to the second moving member, and
wherein the laser beam from the laser light source is emitted in the first direction and is incident on the first deflecting member.

8. The laser microscope apparatus according to Claim 7 further comprising:
a third moving member configured to movably hold the first moving member and move the first moving member in the direction of the optical axis of the objective lens, and
a third deflecting member configured to deflect the laser beam and emit the deflected laser beam in the first direction, the third deflecting member being fixed to the third moving member.
